# EUROPEAN PATENT APPLICATION

(11) **EP 1 219 272 A1**
(43) Date of publication of application: **03.07.2002**
(21) Application number: 00960986.8
(22) Date of filing: 13.09.2000
(51) Int. Cl.: A61F 13/49

(54) **DISPOSABLE DIAPER**

(30) Priority: 21.09.1999 JP 26774399
(71) Applicant: Kao Corporation, Tokyo 103-8210 (JP)
(72) Inventor: YOSHIDA, Satoshi Kao Corporation, Research Labs., Haga-gun Tochigi 321-3426 (JP); MIYAHARA, Satoshi Kao Corporation, Research Labs., Haga-gun Tochigi 321-3426 (JP); ARIMURA, Takahiro Kao Corporation, Research Labs., Haga-gun Tochigi 321-3426 (JP)
(74) Representative: VOSSIUS & PARTNER
(86) International application number: JP0006267
(87) International publication number: WO0121123

(57) **Abstract**

A disposable diaper having a topsheet (2), a backsheet (3) and an absorbent member (4) interposed between two sheets is disclosed. A hot-melt adhesive having a melt viscosity of 10 to 1000 Pa·s at 140°C is used to join any two of members constituting the diaper (1).

## Description

### Technical Field:

The present invention relates to a disposable diaper the constituent members of which, for example, a side flap and a tape tab, are joined together with improved joint strength.

### Background Art:

Disposable diapers of unfolded type or flat type have a tape tab on a side flap disposed on each side of the rear waist portion, with which to fasten the diaper. The tape tab is held between a topsheet and a backsheet which make up the side flaps and fixed therein with a hot-melt adhesive. In recent years breathable materials such as nonwoven fabric are used to make side flaps with increased air-permeability.

Because nonwoven fabric has relatively large interstices among fibers, it is not easy to fixedly join a tape tab to nonwoven fabric with a hot-melt adhesive. It is not easy, in particular, to satisfy both requirements for shear resistance characteristics and for strength characteristics in high-speed 180° peeling, which are of significance for fixing tape tabs. The same problem can arise where a hot-melt adhesive is used to bond other members composing a diaper.

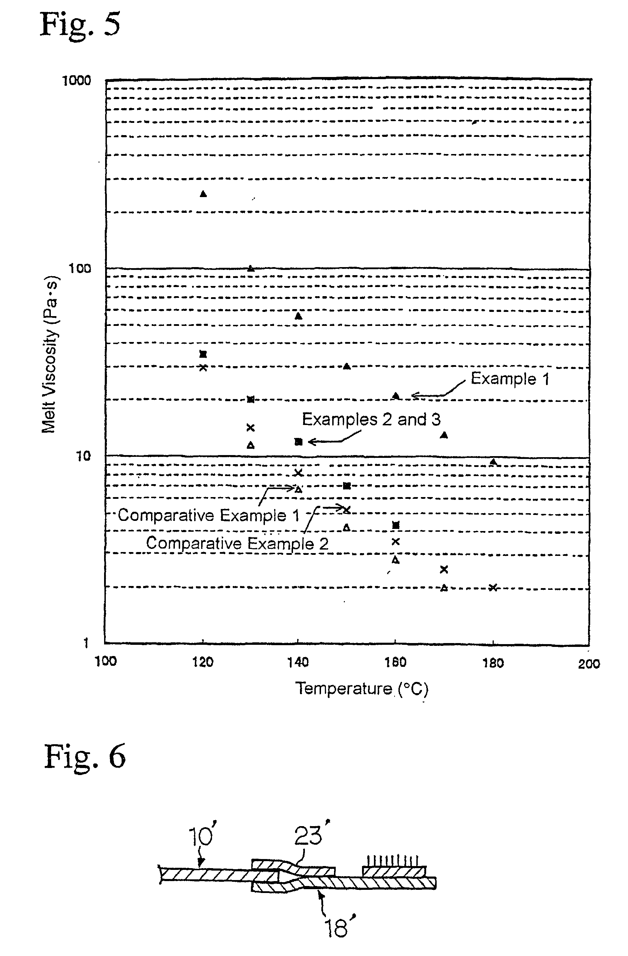

For the purpose of satisfying both the requirements for shear resistance characteristics and high-speed 180° peel strength characteristics, a diaper having tape tabs fixed as shown in Fig. 6 has been proposed, in which one end of a tape tab 18' is fixed to one side of a side flap 10' made of nonwoven fabric, and one end of an auxiliary tape 23' is fixed to the other side of the side flap 10', the other end of the auxiliary tape 23' being fixed to the tape tab 18'. However, this diaper needs an extra material for the auxiliary tape 23', which results in an increase of production cost.

### Disclosure of the Invention:

Accordingly, an object of the present invention is to provide a disposable diaper having improved joint strength between constituent members thereof.

Another object of the present invention is to provide a disposable diaper having improved joint strength between a side flap and a tape tab for fastening the diaper while maintaining air-permeability of the side portions of the diaper.

The present invention accomplishes the above objects by providing a disposable diaper having a liquid-permeable topsheet, a liquid-impermeable backsheet and a liquid-retentive absorbent member interposed between these sheets, wherein a hot-melt adhesive having a melt viscosity of 10 to 1000 Pa·s at 140°C is used to join any two of members constituting the diaper.

The present invention also provides a method for manufacturing a disposable diaper as a preferred method for manufacturing the above-described disposable diaper, which comprises the steps of feeding the above-described hot-melt adhesive contained in a tank of an applicator to an application head through a feed conduit, applying the hot-melt adhesive to a first member constituting the diaper, and then fixedly joining a second member constituting the diaper to the first member, wherein the hot-melt adhesive contained in the tank is melted by heating to a prescribed temperature, and the temperature of the hot-melt adhesive present in the application head is set lower than that of the hot-melt adhesive contained in the tank.

### Brief Description of the Drawings:

Fig. 1 is a perspective showing a disposable diaper according to an embodiment of the present invention being in use.
Fig. 2A is a plan view of the disposable diaper shown in Fig. 1 in its unfolded state, seen from its topsheet side. Figs. 2B and 2C are cross-sections taken along lines I-I and II-II, respectively, of Fig. 2A.
Fig. 3 is a schematic illustration showing a method of measuring a high-speed 180° peel strength.
Fig. 4 schematically illustrates another manner of fixing a tape tab (corresponding to Fig. 2C).
Fig. 5 shows temperature vs. viscosity plots of the hot-melt adhesives used in Examples 1 to 3 and Comparative Examples 1 and 2.
Fig. 6 is a schematic illustration showing a manner of fixing a tape tab in a conventional disposable diaper (corresponding to Fig. 2C).

### Best Mode for Carrying out the Invention:

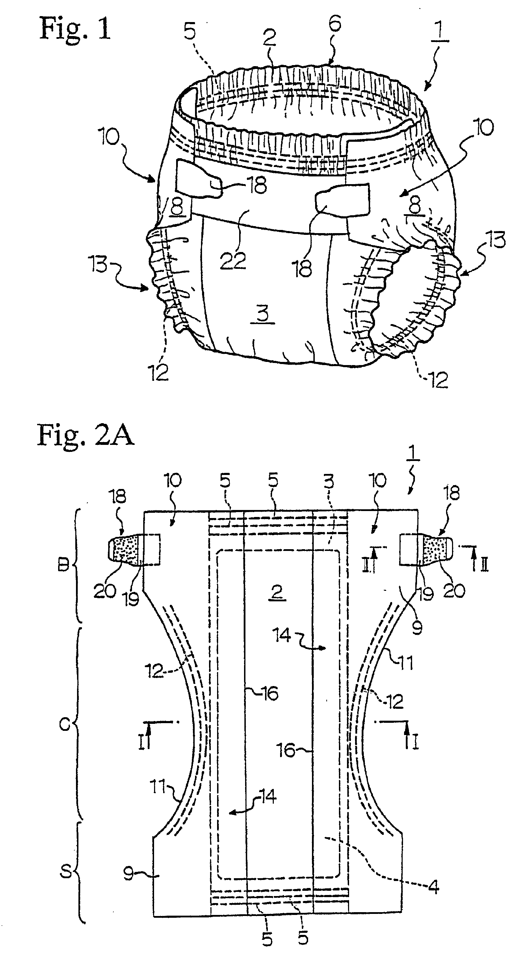

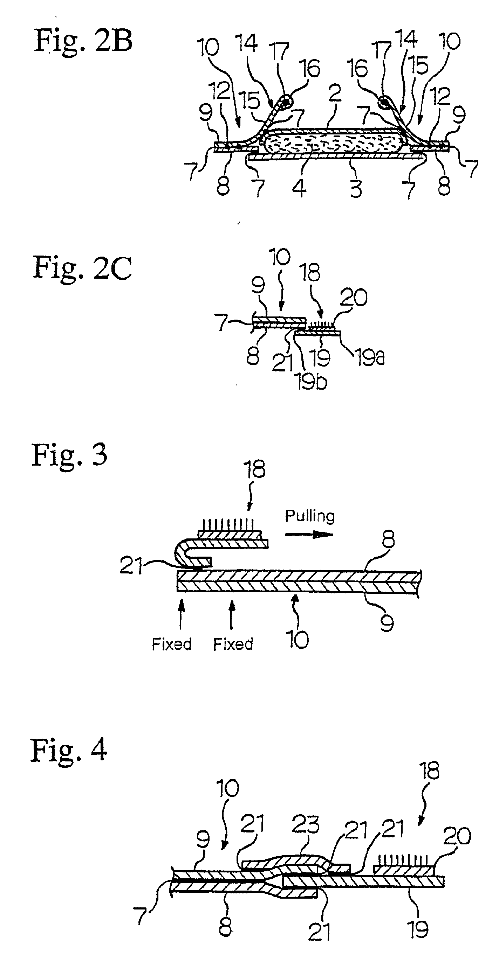

The present invention will be described based on preferred embodiments in practice with reference to the drawings. Fig. 1 is a perspective showing a disposable diaper according to an embodiment of the present invention being in use. Fig. 2 presents a plan view and cross-sectional views of the disposable diaper shown in Fig. 1 in its unfolded state.

The diaper 1 according to this embodiment comprises a crotch portion C which, while worn, is positioned on the crotch of a wearer, a stomach portion S and a back portion B, the stomach portion S and the back portion B being provided in the front and in the rear, respectively, of the crotch portion C and positioned, while worn, on the stomach side and the back side, respectively, of a wearer. The diaper 1 has a liquid-permeable topsheet 2, a liquid-impermeable backsheet 3 and a liquid-retentive absorbent member 4 which is interposed between the sheets 2 and 3. The absorbent member 4 is an oblong rectangle. The topsheet 2 and the backsheet 3 also have an oblong shape and extend from lateral sides and longitudinal ends of the absorbent member 4. In other words, the topsheet 2 and the backsheet 3 have a contour larger than that of the absorbent member. A plurality of elastic members 5, 5, ... are fixed in their stretched state between the topsheet 2 and the backsheet 3 along the lateral direction of the diaper 1 at the longitudinal ends of the diaper 1, whereby waist gathers 6 are formed at the longitudinal ends of the diaper 1.

The topsheet 2 is formed of a liquid-permeable sheet such as nonwoven fabric or perforated film. The backsheet 3 is formed of a liquid-impermeable sheet such as plastic film. The backsheet 3 may be formed of a microporous sheet having water vapor permeability. The backsheet can also be a laminate sheet composed of a liquid-impermeable and water vapor permeable or water vapor impermeable inner sheet and a nonwoven fabric as an outer sheet.

An outer nonwoven fabric 8 is connected to each lateral side of the backsheet 3 with an adhesive 7, such as a hot-melt adhesive, to form an outward extension from each lateral side of the backsheet 3. On the outer nonwoven fabric 8 is superposed an inner nonwoven fabric 9. The nonwoven fabrics 8 and 9 are joined together via an adhesive 7 in such a manner as to form joint areas and non-joint areas. The outer nonwoven fabric 8 and the inner nonwoven fabric 9 joined together constitute a side flap 10 which extends outward from each lateral side of the backsheet 3. Made of nonwoven fabric, the side flaps 10 have air-permeability. As a result, the diaper 1 maintains breathability through its side portions while worn and effectively prevents skin overhydration and a resultant skin rash.

The outer nonwoven fabric 8 may be either a single'layer of nonwoven fabric or a laminate of a plurality of nonwoven fabric layers. Where the outer nonwoven fabric 8 is a laminate, spun-bonded/melt-blown/spun-bonded (SMS) nonwoven fabric, spun-bonded/spun-bonded (SS) nonwoven fabric, etc. can be used as the laminate. It is preferred for the outer nonwoven fabric to have a basis weight of 7 to 30 g/m², particularly 10 to 25 g/m², for securing air-permeability, strength of the joint of a tape tab 18 hereinafter described, and adhesion to a hot-melt adhesive, for preventing a hot-melt adhesive from oozing out while stored for a long time, and from the standpoint of texture, processability and the like. Similarly, the inner nonwoven fabric 9 may be either a single layer of nonwoven fabric or a laminate of a plurality of nonwoven fabric layers. Since the inner nonwoven fabric 9 also functions as an upstanding guard against leaks of body waste as described later, it is desirable for the inner nonwoven fabric 9 to have water repellency. The inner nonwoven fabric 9 preferably has a basis weight of 7 to 30 g/m², particularly 10 to 25 g/m², which is desirable from the viewpoint of air permeability, adhesion to a hot-melt adhesive, prevention of a hot-melt adhesive from oozing during long-term storage, texture, processability, and the like.

The lateral edges 11 of the side flaps 10 are the lateral edges of the diaper 1. The part of each of the lateral edges 11 which is positioned in the crotch portion C is curved inward. A plurality of elastic members 12, 12, ... are fixed in their stretched state between the outer nonwoven fabric 8 and the inner nonwoven fabric 9 along the curve of each lateral edge 11 at the position slightly inward from the lateral edge 11, whereby leg gathers 13 are formed at the crotch portion C of the diaper 1.

The inner nonwoven fabric 9 has an extension 14 which extends inward from the inward edge of the outer nonwoven fabric 8 (i.e., toward the longitudinal center line of the diaper 1). The longitudinal ends of each extension 14 are fixed to the topsheet 2 in such a manner that the extension 14 is inclined inward. The extension 14 has a fixed end 15 and a free end 16. The extension 14 is fixed to the top sheet 2 with an adhesive 9 along the longitudinal direction of the diaper 1 at a position near the inward edge of the outer nonwoven fabric 8 thereby to form the fixed end 15. The free end 16 is formed by the inside end of the inner nonwoven fabric 9 being folded back to form a sleeve. An elastic member 17 is fixed in the sleeve in its stretched state, whereby the extension 14 stands up along the longitudinal direction of the diaper 1 to function as a upstanding guard against leaks of body waste while the diaper 1 is worn.

A tape tab 18 for fastening the diaper is provided on the side flap 10 located on both sides of the back portion B of the diaper 1. The tape tabs 18 each have an oblong shape and are composed of a base sheet 19 made of thick nonwoven fabric or a plastic film and a hook member 20 of a mechanical fastener. The base sheet 19 has a free end 19a and a fixed end 19b on the respective ends in the longitudinal direction. The hook member 20 of a mechanical fastener is fixed near the free end 19a, while the fixed end 19b is fixed to the outer nonwoven fabric 8 of the side flap 10 with a hot-melt adhesive 21. While the diaper 1 is worn, the hook member 20 of the tape tab 18 is engaged with a loop member 22 of a mechanical fastener which is disposed on the backsheet 3 in the stomach portion S.

In the diaper 1 according to the present embodiment, the hot-melt adhesive 21 used to join the base sheet 19 of the tape tab 18 and the outer nonwoven fabric 8 in the side flap 10 has a melt viscosity of 10 to 1000 Pa·s, preferably 10 to 800 Pa·s, still preferably 15 to 500 Pa·s, particularly preferably 30 to 100 Pa·s. Where the tape tab and the nonwoven fabric are bonded with a conventionally employed hot-melt adhesive, it is not easy to satisfy both requirements for shear resistance characteristics and for high-speed 180° peeling strength characteristics simultaneously. Use of a hot-melt adhesive whose melt viscosity falls within the above-recited range makes it possible to satisfy both the requirements. More specifically, if the melt viscosity is less than 10 Pa·s, the requirements for shear resistance characteristics and for high-speed 180° peel strength characteristics are not fulfilled simultaneously. If it exceeds 1000 Pa·s, it is difficult to stably apply the hot-melt adhesive to the base sheet 19. The melt viscosity is measured with a Brookfield viscometer (supplied by Tokyo Keiki K.K.).

The terminology "shear resistance characteristics" as used herein means characteristics of the tape tab hardly peeling from the side flap in shear with a prescribed load applied. The terminology "high-speed 180° peel strength characteristics" as used herein denote characteristics of the tape tab hardly peeling from the side flap when pulled at a high speed in a direction making 180° with the side flap.

As long as the melt viscosity falls within the above-recited range, any hot-melt adhesive can be used with no particular limitation as the adhesive 21. Examples of such hot-melt adhesives are styrene-based hot-melt adhesives and olefin-based hot-melt adhesives. From the standpoint of processability and tack, styrene-based hot-melt adhesives are preferred.

The hot-melt adhesive 21 preferably comprises a base polymer, a tackifier that is solid at ambient temperature, a softener, and an antioxidant.

The base polymer includes styrene-butadiene rubber (SBR), styrene-butadiene-styrene block copolymers (SBS), styrene-isoprene-styrene block copolymers (SIS), styrene-ethylene-butylene-styrene block copolymers (SEBS), and styrene-ethylene-propylene-styrene block copolymers (SEPS). It is preferably used in an amount ranging 30 to 130 parts by weight, particularly 40 to 120 parts by weight, per 100 parts by weight of the total amount of the tackifier and the softener.

The tackifier includes C₅-based petroleum resins, C₉-based petroleum resins, dicyclopentadiene-based petroleum resins, rosin-based petroleum resins, polyterpene resins, and terpenephenol resins. It is preferably used in an amount ranging 60 to 99 parts by weight, particularly 70 to 95 parts by weight, per 100 parts by weight of the total amount of the tackifier and the softener.

The softener includes process oils, mineral oils, various plasticizers, polybutene, liquid tackifying resins, etc. that have a softening point of 10°C or below and an average molecular weight of 200 to 700. It is preferably used in an amount ranging 1 to 40 parts by weight, particularly 5 to 30 parts by weight, per 100 parts by weight of the total amount of the tackifier and the softener.

The antioxidant includes phenol antioxidants, amine antioxidants, phosphorus antioxidants, and benzimidazole antioxidants. It is preferably used in an amount of 0.5 to 3 parts by weight per 100 parts by weight of the total amunt of the base polymer, the tackifier, and the softener.

In addition to the above components, other components generally used in adhesives, such as ultraviolet absorbers, can appropriately be added to the hot-melt adhesive 21.

The hot-melt adhesive 21 is preferably applied at a spread of 20 to 100 g/m², particularly 40 to 80 g/m², to satisfy both the requirements for shear resistance characteristics and high-speed 180° peel strength characteristics and not to ooze out during storage.

The diaper 1 according to the present embodiment is preferably manufactured by the method described hereunder. A hot-melt adhesive 21 contained in a tank of an applicator is fed to an application head through a feed conduit and applied to a fixed end 19b of a base sheet 19 of a tape tab 18. The fixed end 19b is joined and fixed to an outer nonwoven fabric 8 of each side flap 10 to produce a diaper 1. In carrying out the above operation, the hot-melt adhesive 21 contained in the tank is melted by heating to a prescribed temperature, and the temperature of the hot-melt adhesive 21 present in the application head is set lower than that of the hot-melt adhesive contained in the tank, the temperature of the hot-melt adhesive 21 present in the feed conduit being set between the temperature of the hot-melt adhesive 21 present in the application head and that of the hot-melt adhesive 21 contained in the tank.

If the hot-melt adhesive 21 used in the present invention is applied in a traditional manner of applying a hot-melt adhesive, i.e., with the temperatures in the tank, the feed conduit and the application head being equal, the following disadvantages result. In case where the melting temperature of the hot-melt adhesive 21 is low, the hot-melt adhesive 21 does not circulate smoothly in the applicator, imposing a great load on the motor of the circulating pump. Where the melting temperature of the hot-melt adhesive 21 is high, on the other hand, the base sheet 19 of the tape tab 18 suffers thermal damage on contact with the application head. These disadvantages are eliminated by applying the hot-melt adhesive 21 by the above-mentioned method.

In applying the hot-melt adhesive 21, it is preferred that the temperature of the hot-melt adhesive 21 contained in the tank and present in the application head be in a range of from 150 to 200°C and from 120 to 150°C, respectively, particularly of from 160 to 180°C and of from 130 to 140°C, respectively. These ranges are desirable for suppressing thermal deterioration such as carbonization in the tank, reducing the motor load of the circulating pump, securing application stability in the application head, and reducing the thermal damage to the base sheet.

In carrying out application of the hot-melt adhesive 21, it is preferred that the melt viscosity of the hot-melt adhesive 21 contained in the tank and present in the application head be in a range of from 5 to 50 Pa·s and of from 10 to 500 Pa·s, respectively, particularly of from 5 to 40 Pa·s and of from 10 to 300 Pa·s, respectively. These ranges are preferred for reducing the pump load of the applicator and securing application stability of the application head.

The hot-melt adhesive 21 can be applied either in an in-line application system or an off-line application system. Seeing that joining immediately after coating with the hot-melt adhesive generally achieves better joining performance, an in-line application system is preferred.

The present invention is not limited to the above-described embodiments. For example, the position for fixing the tape tab 18 is not limited to the outer nonwoven fabric 8. It can be fixed to the inner nonwoven fabric 9 or between the outer nonwoven fabric 8 and the inner nonwoven fabric 9.

The tape tab 18 being fixed between the outer nonwoven fabric 8 and the inner nonwoven fabric 9, an auxiliary tape 23 may be used to fix the inner nonwoven fabric 9 and the tape tab 18 as shown in Fig. 4. However, the hot-melt adhesive used in the present invention exhibits sufficient performance without the aid of such an auxiliary tape 23 because of the high joint strength to the nonwoven fabrics 18 and 19 and the high peel strength between the tape tab 18 and the nonwoven fabrics 18 and 19 as demonstrated in Examples hereinafter given. Thus, the disposable diaper of the present invention also brings the advantage of cost reduction. In this case, fixing of the auxiliary tape 23 to the inner nonwoven fabric 9 and the tape tab 18 is preferably made with the above-described hot-melt adhesive 21. Use of the auxiliary tape 23 is advantageous in case a diaper is not designed to secure a sufficient joint area between the outer nonwoven fabric 8 or the inner nonwoven fabric 9 and the tape tab 18.

The hook member 20 of the mechanical fastener may be replaced with an adhesive coating layer. In this case, the loop member 22 of the mechanical fastener which is provided on the backsheet 3 in the diaper stomach portion S is replaced with a plastic film to which the adhesive coating layer can be stuck.

Combinations of diaper constituent members that can be joined together with the above-described hot-melt adhesive 21 include, in addition to the combination of the side flaps 10 and the tape tabs 18, a combination of the outer nonwoven fabric 8 and the inner nonwoven fabric 9, a combination of the backsheet 3 and the outer nonwoven fabric 8, and a combination of the topsheet 2 and the backsheet 3.

### EXAMPLES

The present invention will now be illustrated in greater detail, but the scope of the present invention is by no means limited thereto.

### EXAMPLES 1 TO 3 AND COMPARATIVE EXAMPLES 1 AND 2

Disposable diapers shown in Figs. 1 and 2 were made. The melt viscosities (at 140°C) of the hot-melt adhesives used to fix a tape tab to the side flaps are shown in Table 1. Table 1 also includes the kinds of the outer nonwoven fabrics which made the side flaps. The tape tab used had a spun-bonded nonwoven fabric made of polypropylene as a base sheet.

In carrying out hot-melt adhesive application in Examples 1 and 2, the temperatures and the melt viscosities of the hot-melt adhesives contained in the tank of the applicator and present in the application head were 160 to 180°C and 130 to 140°C, respectively, and 5 to 20 Pa·s and 12 to 100 Pa·s, respectively. The hot-melt adhesive used in Example 3 was the same as used in Example 2.

In Comparative Examples 1 and 2, all the temperatures of the hot-melt adhesives contained in the tank of the applicator, present in the feed conduit and present in the application head were set at 140°C. The melt viscosities of the hot-melt adhesives at 140°C were 7 Pa·s (Comparative Example 1) and 8 Pa·s (Comparative Example 2).

The diapers obtained in Examples and Comparative Examples were evaluated for shear resistance characteristics and high-speed 180° peel strength characteristics by the following methods. The results obtained are shown in Table 1. In Fig. 5 are shown temperature vs. viscosity plots of the hot-melt adhesives used in Examples 1 to 3 and Comparative Examples 1 and 2. The viscosity of the hot-melt adhesives was measured with a Brookfield viscometer (supplied by Tokyo Keiki K.K.). Measurement was made by using a rotor No. 4 at 6 rpm.

### Shear resistance characteristics:

The tape tab was set vertically, and a 9.8 N load was applied to its free end in an environment of 40°C. The time required for the tape tab to separate from the side flap in shear was measured.

### High-speed 180° peel strength characteristics:

The tape tab was pulled at a peel angle of 180° to the side flap as shown in Fig. 3 at a speed of 100m/min, and the maximum load was measured. In Table 1, "material failure" means that the tape tab is not peeled off the side flap but causes the side flap to be destroyed near the joint in the measurement.

**TABLE 1**

| | Melt Viscosity (140°C) of Hot-melt Adhesive (Pa·s) | Outer Nonwoyen Fabric Making Side Flap | Shear Resistance Characteristics (min) | High-speed 180° Peel Strength Characteristics (N/30mm) |
|---|---|---|---|---|
| Example 1 | 56 | SMS; 15 g/m² | 65.4 | material failure |
| Example 2 | 12 | SMS; 15 g/m² | 38.5 | material failure |
| Example 3 | 12 | SMS; 8 g/m² | 31.7 | material failure |
| Comparative Example 1 | 7 | SMS; 15 g/m² | 5 | material failure |
| Comparative Example 2 | 8 | SMS; 15 g/m² | 60 | 4.9 |

As is apparently understood from the results in Table 1, the disposable diapers of Examples in which the tape tab was fixed to the side flaps with a hot-melt adhesive having a specific melt viscosity satisfy both the requirements for shear resistance characteristics and high-speed 180° peel strength characteristics. To the contrary, the disposable diaper of Comparative Examples satisfies the requirement for high-speed 180° peel strength characteristics but with insufficient shear resistance characteristics or, vise versa, it satisfies the requirement for shear resistance characteristics but has insufficient high-speed 180° peel strength characteristics. While not described in Table, none of the diapers prepared in Examples showed thermal damage to their tape tabs from the hot-melt adhesive applied.

In Example 3, although both the requirements for shear resistance characteristics and high-speed 180° peel strength characteristics are fulfilled, some of the samples stored in an environment of 40°C and 80% RH for 1 month slightly suffered from oozing of the hot-melt adhesive. Such oozing was not observed in Examples 1 and 2.

For reference, when an auxiliary tape was used to fix the tape tab in Comparative Example 1, the diaper exhibited the same level of shear resistance characteristics as that of the diaper of Example 1.

### Industrial Applicability:

The present invention provides a disposable diaper having improved joint strength between constituent members thereof. In particular, where side flaps are made of nonwoven fabric, the joint strength between the side flaps and the tape tabs is increased while retaining the breathability of the side flaps. In this embodiment, since sufficient joint strength is secured without the aid of an auxiliary tape, the material to be used can be saved, which leads to a reduced production cost. Besides, the amount of the backsheet can be reduced compared with conventional diapers, which also leads to a reduction in production cost.

## Claims

1. A disposable diaper having a liquid-permeable topsheet, a liquid-impermeable backsheet and a liquid-retentive absorbent member interposed between these sheets, wherein a hot-melt adhesive having a melt viscosity of 10 to 1000 Pa·s at 140°C is used to join any two of members constituting the diaper.

2. The disposable diaper according to claim 1, wherein a side flap is provided on each side of a back portion of the diaper which is, while worn, positioned in the back of a wearer, a tape tab for fastening the diaper is provided on each side flap, said side flap is made of nonwoven fabric, and said tape tab is fixedly joined to said side flap with said hot-melt adhesive.

3. The method for manufacturing a disposable diaper according to claim 1, which comprises the steps of feeding said hot-melt adhesive contained in a tank of an applicator to an application head through a feed conduit, applying said hot-melt adhesive to a first member constituting the diaper, and then fixedly joining a second member constituting the diaper to said first member, wherein
said hot-melt adhesive contained in said tank is melted by heating to a prescribed temperature, and the temperature of said hot-melt adhesive present in said application head is set lower than that of said hot-melt adhesive contained in said tank.

4. The method for manufacturing a disposable diaper according to claim 3, wherein the temperature of said hot-melt adhesive contained in said tank and present in said application head is from 150 to 200°C and from 120 to 150°C, respectively.

5. The method for manufacturing a disposable diaper according to claim 3, wherein the melt viscosity of said hot-melt adhesive contained in said tank and present in said application head is from 5 to 50 Pa·s and from 10 to 500 Pa·s, respectively.
